# EUROPÄISCHE PATENTSCHRIFT

(11) **EP 1 595 877 B1**
(45) Veröffentlichungstag und Bekanntmachung des Hinweises auf die Patenterteilung: **11.03.2009**
(21) Anmeldenummer: 05009474.7
(22) Anmeldetag: 29.04.2005
(51) Int. Cl.: C07D 317/46

(54) **Verfahren zur Herstellung von 5-Brom-2,2-Difluorbenzo-(1,3)-Dioxolen**
Process for the preparation of 5-bromo-2,2-difluorobenzo-(1,3)-dioxoles
Procédé de préparation de 5-bromo-2,2-difluorobenzo-(1,3)-dioxolanes

(30) Priorität: 14.05.2004 DE 102004024012
(43) Veröffentlichungstag der Anmeldung: 16.11.2005
(73) Patentinhaber: Saltigo GmbH, 40764 Langenfeld (DE)
(72) Erfinder: Pleschke, Axel, Dr., 51069 Köln (DE); Marhold, Albrecht, Dr., 51373 Leverkusen (DE)
(74) Vertreter: Wichmann, Birgid

(56) Entgegenhaltungen:
- WO-A-93/24483
- CHERRY G. ET AL.: "Elecrophilic bromination of phenol ethers in superacid solution using alkali bromide" TETRAHEDRON LETTERS, Bd. 31, Nr. 14, 1990, Seiten 2007-2010, XP002344442

## Beschreibung

Die Erfindung betrifft ein Verfahren zur Herstellung von 5-Brom-2,2-difluorbenzo-[1,3]-dioxolen sowie deren Verwendung zur Herstellung von Arzneimitteln und Pflanzenschutzmitteln.

5-Brom-2,2-difluorbenzo-[1,3]-dioxole spielen eine wichtige Rolle als Vorprodukte für agrochemische und pharmazeutische Wirkstoffe, siehe auch EP 333 658 A und WO 93/24483.

Die Bromierung von 2,2-Difluorbenzo-[1,3]-dioxol mittels FeCl₃ oder CuCl₃ ist aus WO 93/24483 bekannt. Weiterhin ist die Bromierung von Phenolethern unter Verwendung von HF-SbF₅-Gemischen bekannt (Cherry et al., Tetrahedron Lett., 31, 1990, 2007-2010).

Bislang wurden zur Herstellung von 5-Brom-2,2-difluorbenzo-[1,3]-dioxolen nur unbefriedigende Methoden offenbart. So beschreibt US 4,895,871 eine Bromierung von 2,2-Difluorbenzo-[1,3]-dioxol mit elementarem Brom in toxischem Tetrachlorkohlenstoff. Die Ausbeute ist weiterhin mit 50 % sehr gering, so dass dieses Verfahren für industrielle Zwecke nicht in Betracht kommt.

Es bestand also der Bedarf nach einem Verfahren, das die Herstellung von 5-Brom-2,2-difluorbenzo-[1,3]-dioxolen in technischem Maßstab in einfacher Weise und in guten Ausbeuten ermöglicht.

Es wurde nun ein Verfahren zur Herstellung von Verbindungen der Formel (I) gefunden, 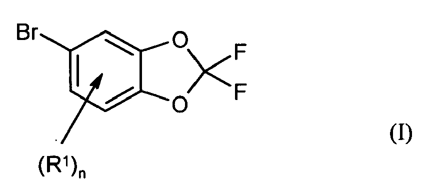 in der
- R¹: für C₁-C₄-Alkyl, C₁-C₄-Alkoxy, wobei die Alkyl- bzw Alkoxyreste gegebenenfalls durch C₁-C₄-Alkoxy-Reste weiter substituiert sein können, Brom, Chlor oder Fluor und
- n: für 0 oder 1 steht
das **dadurch gekennzeichnet** ist, **dass** Verbindungen der Formel (II) in Gegenwart von mindestens zwei Friedel-Crafts-Katalysatoren von denen mindestens einer BF₃, BCl₃, FeCl₃, FeBr₃, SbCl₃, ZnCl₂, ZnBr₂, TiCl₂, TiCl₄, TiBr₄, ZrCl₄, MoBr₄, MoO₃, CuCl₂ oder Cu₂Cl₂ ist und einer Fluorwasserstoff ist, mit Brom umgesetzt werden.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

**Alkyl** beziehungsweise **Alkoxy** steht jeweils unabhängig für einen geradkettigen, cyclischen, verzweigten oder unverzweigten Alkyl- beziehungsweise Alkoxy-Rest, wobei die genannten Reste gegebenenfalls durch C₁-C₄-Alkoxy-Reste weiter substituiert sein können.

C₁-C₄-Alkyl steht beispielsweise und bevorzugt für Methyl, Ethyl, n-Propyl, iso-Propyl, oder tert.-Butyl.

C₁-C₄-Alkoxy steht beispielsweise und bevorzugt für Methoxy, Ethoxy, n-Propoxy, iso-Propoxy und n-Butoxy.

Im Folgenden werden die bevorzugten Substitutionsmuster definiert:
- R¹: steht besonders bevorzugt für Methyl, Ethyl, n-Propyl, Chlor, Fluor und Brom, ganz besonders bevorzugt für Chlor oder Fluor.
- n: steht bevorzugt für 0 oder 1 besonders bevorzugt für 0.

Bevorzugte Verbindungen der Formel (II) sind 2,2-Difluorbenzo-[1,3]-dioxol und 5-Chlor-2,2-difluorbenzo-[1,3]-dioxol.

Bevorzugte Verbindungen der Formel (II) sind 5-Brom-2,2-difluorbenzo-[1,3]-dioxol und 6-Chlor-5-brom-2,2-difluorbenzo-[1,3]-dioxol.

Das erfindungsgemäße Verfahren wird in Gegenwart von mindestens zwei Friedel-Crafts-Katalysatoren von denen einer Fluorwasserstoff ist durchgeführt. Dabei ist für den Fachmann klar, dass sich die beiden Komponenten zu Protonensäuren verbinden können, ohne dass dies jeweils gesondert erwähnt wird.

Geeignete Friedel-Crafts-Katalysatoren neben Fluorwasserstoff sind beispielsweise Lewissäuren wie BF₃, BCl₃, FeCl₃, FeBr₃, SbCl₃, SbBr₃, SnCl₄, ZnCl₂, ZnBr₂, TiCl₂, TiCl₄, TiBr₄, ZrCl₄, MoBr₄, MoO₃, CuCl₂, Cu₂Cl₂. Bevorzugte Friedel-Crafts-Katalysatoren sind BF₃, BCl₃, FeCl₃, SnCl₄, ZnCl₂, TiCl₄ und MoO₃, weiter bevorzugt sind BF₃, BCl₃, TiCl₄ und MoO₃, noch weiter bevorzugt ist TiCl₄.

Das molare Verhältnis von Friedel-Crafts-Katalysatoren, die nicht Fluorwasserstoff sind, zu Verbindungen der Formel (I) kann beispielsweise 0,1 bis 10 betragen, bevorzugt jedoch 0,1 bis 3 und besonders bevorzugt 0,5 bis 2. Größere Mengen sind möglich, jedoch unwirtschaftlich.

Das molare Verhältnis von Fluorwasserstoff zu Verbindungen der Formel (I) kann beispielsweise 0,1 bis 1000 betragen, bevorzugt jedoch 1 bis 10 und besonders bevorzugt 1,5 bis 5. Größere Mengen sind möglich, jedoch unwirtschaftlich.

Das molare Verhältnis von Brom zu Verbindungen der Formel (I) kann beispielsweise 0,5 bis 1,2 betragen. Größere Mengen sind möglich, gehen jedoch mit sinkender Selektivät für monobromierte Produkte einher.

Die Reaktionstemperatur kann beispielsweise -30 bis 90°C betragen, bevorzugt -20 bis 80°C, besonders bevorzugt 0 bis 20°C, der Reaktionsdruck 0,5 bis 100 bar, bevorzugt 0,9 bis 12 bar.

Die Dosierabfolge zwischen Verbindung der Formel (I), Friedel-Crafts-Katalysatoren und Brom ist beliebig, aus Selektivitätsgründen wird jedoch vorzugsweise Brom zuletzt zugegeben.

Die Reaktion sollte aufgrund der korrodierenden Wirkung von Fluorwasserstoff in Edelstahl- oder Monelvorrichtungen durchgeführt werden.

Vorzugsweise erfolgt die Aufarbeitung dadurch, dass zunächst überschüssiges Brom, Fluorwasserstoff und gegebenenfalls Friedel-Crafts-Katalysator abdestilliert oder durch Phasentrennung abgetrennt werden und das verbleibende Produkt gegebenenfalls durch Kristallisation oder Destillation gereinigt wird. Das ursprünglich abgetrennte Gemisch aus überschüssigem Brom, Fluorwasserstoff und gegebenenfalls Friedel-Crafts-Katalysator kann ohne weitere Aufarbeitung erneut in die Reaktion zurückgeführt werden.

Auf erfindungsgemäße Weise können Verbindungen der Formel (I) in hoher Reinheit und Ausbeute in einfacher Weise erhalten werden.

Die erfindungsgemäß herstellbaren Verbindungen der Formel (I) eignen sich insbesondere zur Anwendung in einem Verfahren zur Herstellung von Arzneimitteln, Agrochemikalien oder Zwischenprodukten davon.

### Beispiele:

### Beispiel 1

In einem Edelstahlautoklaven werden 95 ml Fluorwasserstoff, 14,8 g Titantetrachlorid und 300 g 5-Chlor-2,2-difluorbenzodioxol bei 0 °C vorgelegt. Nachdem die Mischung für 20 Minuten gerührt wurde, werden 249 g Brom bei 0 -10 °C zudosiert. Der entstehende Bromwasserstoff wird bei 0,5 bar über einen auf -15 °C gekühlten Kondensator entspannt. Nach Ende der Zugabe wird noch 1 Stunde bei 10 °C und 1 Stunde bei 20°C nachgerührt. Anschließend wird bei einem leichten Vakuum der Fluorwasserstoff abdestilliert und kondensiert. Der Rückstand wird auf 900 g Eis abgelassen und mit Dichlormethan nach Schmelzen des Eises aufgenommen. Die Dichlormethanlösung wird mit Bicarbonatlösung gewaschen, getrocknet und destilliert. Nach einem kleinen Vorlauf mit 5-Chlor-2,2-difluorbenzodioxol erhält man 312 g 5-Brom-6-chlor-2,2-difluorbenzodioxol mit einem Siedepunkt von 95 °C/16 mbar. Die Ausbeute bezogen auf umgesetztes 5-Chlor-2,2-difluorbenzodioxol beträgt 88,7 %.

¹H-NMR (400 MHz, CDCl₃): 7,32 (s, 1H), 7,19 (s, 1H)

### Beispiel 2

Analog Beispiel 1 werden 300 g Difluorbenzodioxol in 115 ml Fluorwasserstoff in Gegenwart von 18 g Titantetrachlorid mit 303 g Brom umgesetzt. Nach Aufarbeitung werden 380 g 5-Brom-2,2-difluorbenzodioxol erhalten; Siedepunkt 74-75 °C/16 mbar. Ausbeute: 62 % d.Th.

Bei nochmaliger Durchführung konnte eine Ausbeute von 70 % erzielt werden.

## Patentansprüche

1. Verfahren zur Herstellung von Verbindungen der Formel (I), 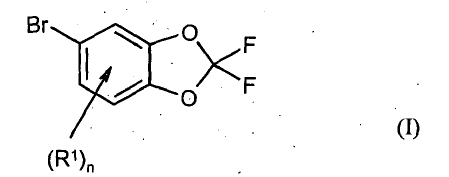 in der
R¹ für C₁-C₄-Alkyl, C₁-C₄-Alkoxy, wobei die Alkyl bzw. Alkoxyreste gegebenenfalls durch C₁-C₄-Alkoxy-Reste weiter substituiert sein können, Brom, Chlor oder Fluor und
n für 0 oder 1 steht,
**dadurch gekennzeichnet, dass** Verbindungen der Formel (II) 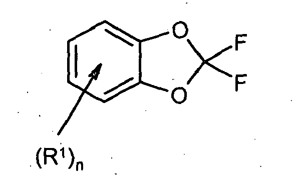 in Gegenwart von mindestens zwei Friedel-Crafts-Katalysatoren von denen mindestens einer BF₃, BCl₃, FeCl₃, FeBr₃, SbCl₃, ZnCl₂, ZnBr₂, TiCl₂, TiCl₄, TiBr₄, ZrCl₄, MoBr₄, MoO₃, CuCl₂ oder Cu₂Cl₂ ist und einer Fluorwasserstoff ist, mit Brom umgesetzt werden.

2. Verfahren nach Anspruch 1, **dadurch gekennzeichnet, dass** R¹ in Formel (I) für Methyl, Ethyl, n-Propyl, Chlor, Fluor und Brom und n für 0 oder 1 steht.

3. Verfahren nach Anspruch 1 oder 2, **dadurch gekennzeichnet, dass** das molare Verhältnis von Friedel-Crafts-Katalysatoren, die nicht Fluorwasserstoff sind zu Verbindungen der Formel (I) 0,1 bis 10 beträgt.

4. Verfahren nach einem oder mehreren Ansprüchen 1, bis 3, **dadurch gekennzeichnet, dass** das molare Verhältnis von Fluorwasserstoff zu Verbindungen der Formel (I) 0,1 bis 1000 beträgt.

5. Verfahren nach einem oder mehreren Ansprüchen 1, bis 4, **dadurch gekennzeichnet, dass** das molare Verhältnis von Brom zu Verbindungen der Formel (I) 0,5 bis 1,2 beträgt.

6. Verfahren nach einem oder mehreren Ansprüchen 1 bis 5, **dadurch gekennzeichnet, dass** bei der Dosierabfolge zwischen Verbindung der Formel (I), Friedel-Crafts-Katalysatoren und Brom, Brom zuletzt zugegeben wird.

## Claims

1. Process for preparing compounds of the formula (I) 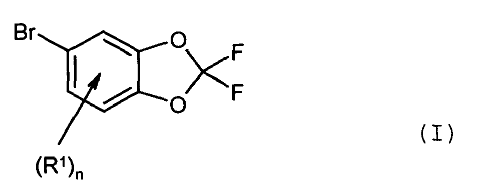 in which
R¹ is C₁-C₄-alkyl, C₁-C₄-alkoxy, where the alkyl and alkoxy radicals may optionally be further substituted by C₁-C₄-alkoxy radicals, bromine, chlorine or fluorine and
n is 0 or 1,
**characterized in that** compounds of the formula (II) 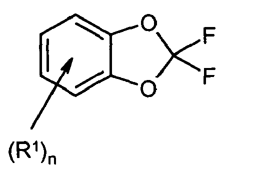 are reacted with bromine in the presence of at least two Friedel-Crafts catalysts of which at least one is BF₃, BCl₃, FeCl₃, FeBr₃, SbCl₃, ZnCl₂, ZnBr₂, TiCl₂, TiCl₄, TiBr₄, ZrCl₄, MoBr₄, MoO₃, CuCl₂ or Cu₂Cl₂ and one is hydrogen fluoride.

2. Process according to Claim 1, **characterized in that** R¹ in formula (I) is methyl, ethyl, n-propyl, chlorine, fluorine and bromine, and n is 0 or 1.

3. Process according to Claim 1 or 2, **characterized in that** the molar ratio of Friedel-Crafts catalysts which are not hydrogen fluoride to compounds of the formula (I) is 0.1 to 10.

4. Process according to one or more of Claims 1 to 3, **characterized in that** the molar ratio of hydrogen fluoride to compounds of the formula (I) is 0.1 to 1000.

5. Process according to one or more of Claims 1 to 4, **characterized in that** the molar ratio of bromine to compounds of the formula (I) is 0.5 to 1.2.

6. Process according to one or more of Claims 1 to 5, **characterized in that,** in the metering sequence between compound of the formula (I), Friedel-Crafts catalysts and bromine, bromine is added last.

## Revendications

1. Procédé pour la préparation de composés de formule (I) 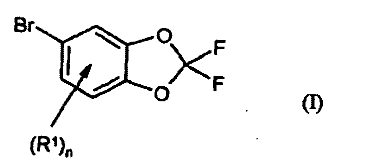 dans laquelle
R¹ représente C₁-C₄-alkyle, C₁-C₄-alcoxy, où les radicaux alkyle ou alcoxy peuvent le cas échéant être substitués davantage par des radicaux C₁-C₄-alcoxy, ou R¹ représente brome, chlore ou fluor et
n vaut 0 ou 1,
**caractérisé en ce qu'**on transforme des composés de formule (II) 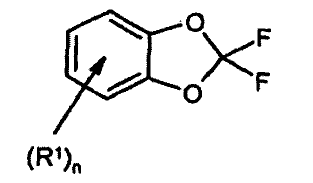 en présence d'au moins deux catalyseurs de Friedel-Craft, dont au moins un est BF₃, BCl₃, FeCl₃, FeBr₃, SbCl₃, ZnCl₂, ZnBr₂, TiCl₂, TiCl₄, TiBr₄, ZrCl₄, MoBr₄, MoO₃, CuCl₂ ou Cu₂Cl₂ et un est l'acide fluorhydrique, avec du brome.

2. Procédé selon la revendication 1, **caractérisé en ce que** R¹ dans la formule (I) représente méthyle, éthyle, n-propyle, chlore, fluor et brome et n vaut 0 ou 1.

3. Procédé selon la revendication 1 ou 2, **caractérisé en ce que** le rapport molaire des catalyseurs de Friedel-Craft, qui ne sont pas l'acide fluorhydrique, aux composés de formule (I) est de 0,1 à 10.

4. Procédé selon l'une ou plusieurs des revendications 1 à 3, **caractérisé en ce que** le rapport molaire de l'acide fluorhydrique aux composés de formule (I) est de 0,1 à 1000.

5. Procédé selon l'une ou plusieurs des revendications 1 à 4, **caractérisé en ce que** le rapport molaire du brome aux composés de formule (I) est de 0,5 à 1,2.

6. Procédé selon l'une ou plusieurs des revendications 1 à 5, **caractérisé en ce que** dans l'ordre de dosage entre le composé de formule (I), les catalyseurs de Friedel-Craft et le brome, le brome est ajouté en dernier lieu.
